# EUROPEAN PATENT APPLICATION

(11) **EP 4 212 594 A1**
(43) Date of publication of application: **19.07.2023**
(21) Application number: 23151652.7
(22) Date of filing: 16.01.2023
(51) Int. Cl.: C09B 69/10, G01N 33/52, G01N 33/58

(54) **WATER-SOLUBLE -CONJUGATED FLUORESCENT 1,1' -BINAPHTHYL-BASED TANDEM POLYMERS**

(30) Priority: 17.01.2022 EP 22151897
(71) Applicant: Miltenyi Biotec B.V. & Co. KG, 51429 Bergisch Gladbach (DE)
(72) Inventor: Dose, Christian, 51429 Bergisch Gladbach (DE); Jenning, Travis, 51429 Bergisch Gladbach (DE); Berndt, Daniel, 51429 Bergisch Gladbach (DE); Meineke, Dirk, 51429 Bergisch Gladbach (DE)
(74) Representative: Kisters, Michael Marcus

(57) **Abstract**

The invention is directed to a conjugate having the general formula (I)

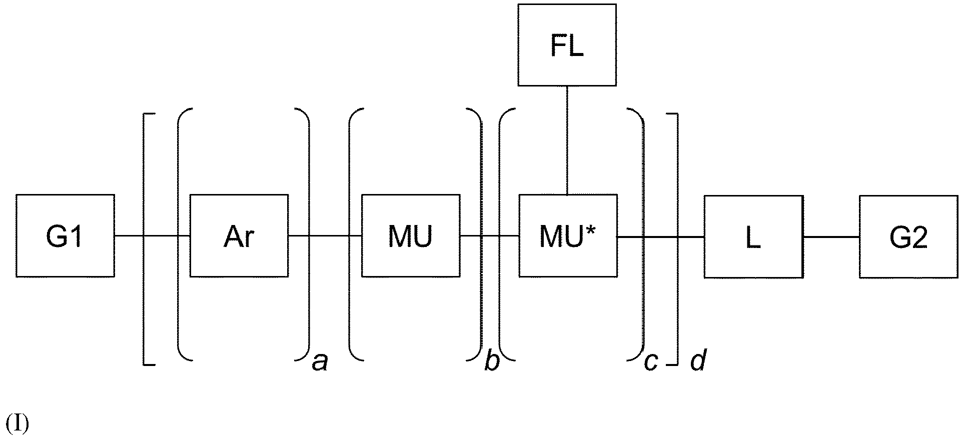

Wherein AR, MU and MU* are repeating units of a polymer and MU and MU* are polymer modifying units or band gap modifying units which are evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 0.1 to 90 mol%
d is 1 to 10 000; with the provisio that a + b + c = 100 mol%
**characterized in that** AR is connected in the polymer chain via the 2,2' or 3,3' or 4,4' or 5,5' or 6,6' or 7,7' or 8,8' positions according to general formula (II)

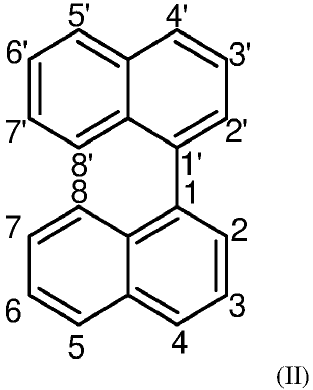

Wherein the remaining positions 2,2'; 3,3 ; 4,4'; 5,5 ; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COCI, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)ₓ(OCH₂CH₂)_{y}O(CH₂)_{z}CH₃, wherein x is an integer from 0 to 20; y is an integer from 0 to 50 and z is an integer from 0 to 20.

## Description

The present invention is to water-soluble fluorescent polymer dyes and their use in conjugates with antibodies for cell detection and analysis.

### BACKGROUND

Immunofluorescent labeling is important for the detailed analysis and specific isolation of target cells from a biological specimen in both, research and clinical applications. The workflow for these methods generally employ a fluorescent tag attached to a targeting molecule which is then used to identify an analyte of interest in the biological sample. Both the sensitivity toward analyte detection and the ability to multiplex many analytes simultaneously are highly sought-after features in cellular analysis workflows. The sensitivity of the analysis largely depends on the brightness of a fluorescent tag, while the ability to multiplex the detection of many analytes simultaneously relies upon the discriminate optically between the different fluorescent tags. Water-soluble π-conjugated polymers have the potential to provide a solution to both sensitivity and multiplexed analysis.

Pi-conjugated polymers as fluorescent probes have become a mainstay in the toolbox of fluorescence detection of biologically-relevant analytes. Due to their strong interaction and ability to absorb light, combined with their bright fluorescence intensity, fluorescent polymers have an enhanced ability to increase the sensitivity of analyte detection over previous technologies. The current trend in the detection of biological analytes has been placing an increased demand on fluorescent systems to have color-tuneable properties, such that individual fluorescence colors may be used to enable multiplexed analyte detection. To be useful in bio-analyte detection applications, therefore, a fluorescent system must have controllable design, such that both absorption and emission may be tuned separately to both the desired excitation and emission. A fluorescent system capable of enabling highly-sensitive multiplexed detection of biomolecular analytes combining highly tuneable excitation energy and, separately, highly-tuneable emission energy within the same system does not exist.

The ideal π-conjugated polymer suited for this problem would natively have a high-energy bandgap allowing it's excitation by ultraviolet (UV) light. At the same time it should be easily possible to red-shift the excitation energy across the visible spectrum through the use of band gap modifying units. Furthermore, it should be possible to separately tune the fluorescence emission energy of such a polymer through the integration of fluorescent energy acceptor dyes. These acceptor dyes may be used to funnel energy from the polymer backbone and yield fluorescent light at a unique wavelength for detection in a color-tuneable manner. In this way, the π-conjugated polymer would have the ability to individually control the excitation wavelength so that it overlaps with common lasers of excitation (eg. Lasers with 355 nm, 405 nm, 488 nm, 561 nm, or 640 nm lines), while simultaneously but separately tuning the wavelength of fluorescence emission. This color-tuneable π-conjugated polymer system would be of tremendous use toward the challenge of multiplexed analyte detection of biomolecules.

Such water-soluble π-conjugated polymers are for example described in "Synthesis of Binaphthyl-Oligothiophene Copolymers with Emissions of Different Colors: Systematically Tuning the Photoluminescence of Conjugated Polymers" by K. Y. Musick, Q.-S. Hu, L. Pu, Macromolecules 1998, 31, 2933-2942.

Further,US20180009990A1 and US 10604657B2 mention 4,4'-polymerized binaphthyl substituted with PEGs in the 2,2'positions. This references a binapthyl-based polymer in which the 4,4' bond is attached to neighboring monomers via polyparaethyniline (PPE) bond, which utilizes sp³ hybridized orbitals. The binaphthyl-based polymers disclosed in this invention are bound directly to neighboring co-monomers without ethyniline bonding, resulting in sp² hybridized bonding and fully-aromatic conjugated systems. Water solvated polymeric dyes and polymeric tandem dyes are provided. The polymeric dyes include a water solvated light harvesting multichromophore having a conjugated segment of aryl or heteroaryl co-monomers linked via covalent bonds, vinylene groups or ethynylene groups. The polymeric tandem dyes further include a signaling chromophore covalently linked to the multichromophore in energy-receiving proximity therewith.

US10481161B2 discloses 4,4'-polymerized binaphthyl substituted with substituents in the 2,2' position. Water solvated polymeric dyes and polymeric tandem dyes are provided. The polymeric dyes include a water solvated light harvesting multi chromophore having a conjugated segment of aryl or heteroaryl co-monomers linked via covalent bonds, vinylene groups or ethynylene groups. The polymeric tandem dyes further include a signaling chromophore covalently linked to the multi chromophore in energy-receiving proximity therewith.

Object of the invention was to provide dyes which can be tuned to a desired excitation energy by choosing a modifying unit (MU).

### BRIEF DESCRIPTION OF THE INVENTION

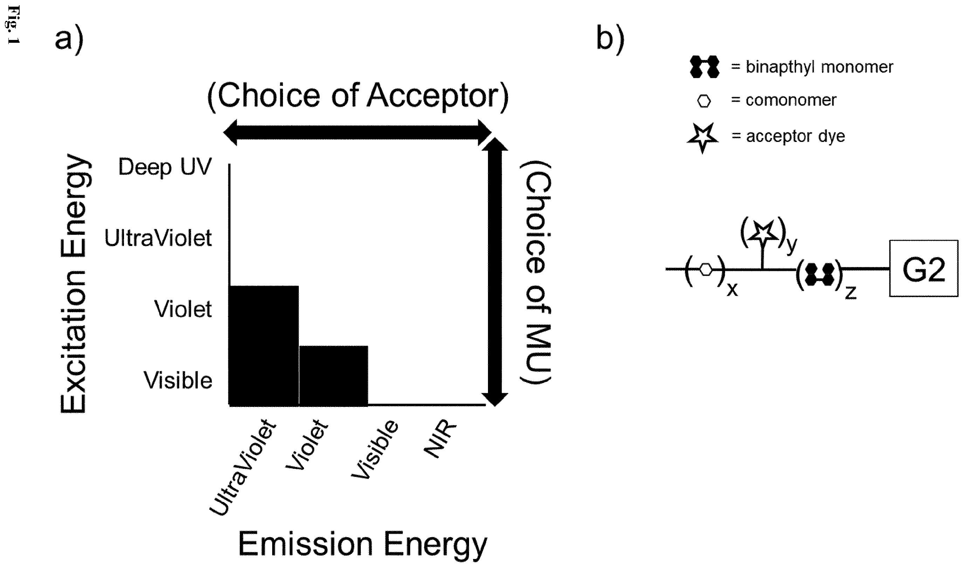

Fig. 1 shows a scheme to demonstrate how the Poly-binaphthyl-co-monomer tandem system may be used to separately tune the excitation energy through the choice of modifying unit (MU) and the emission energy through the choice of Acceptor dye. In this way, the invention may be used to create a color-tunable state of matter in which excitation and emission may be individually controlled. The black boxes represent combinations which are not possible. b) Graphical representation of a copolymer binaphthyl tandem system bound to a biological moiety (G2) in which choice of comonomer (x) allows the tuning of the excitation energy of the polymer, choice of tandem dye (y) allows the tuning of the emission energy of the system, and choice of binaphthyl monomer (z) with different bonding between monomers allows tuning of the efficiency of energy transfer from the polymer backbone to the acceptor dye.

Accordingly, an object of the invention is a conjugate having the general formula (I) 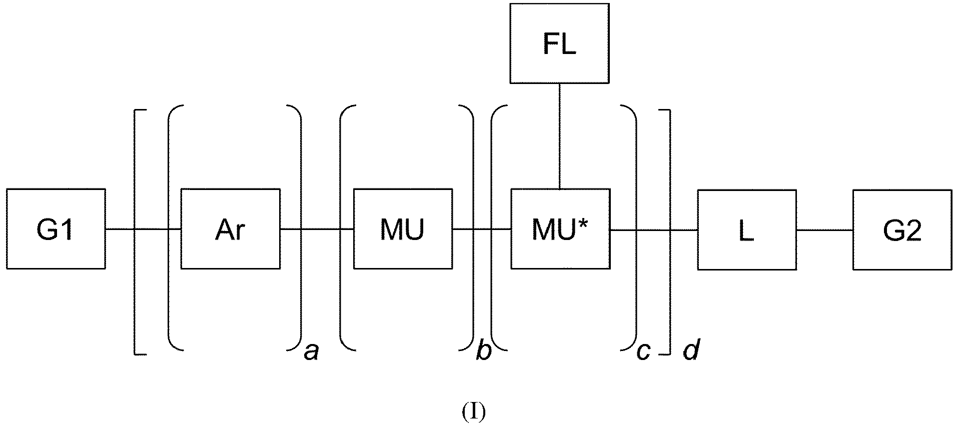
Wherein AR, MU and MU* are repeating units of a polymer
MU and MU* are polymer modifying units or band gap modifying units which are evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 0.1 to 90 mol%, preferable 1-80 mol%, more preferable 5 to 70 mol % and most preferable 10 to 60 mol%,
d is 1 to 10 000; with the provisio that a + b + c = 100 mol%
**characterized in that** AR is connected in the polymer chain via the 2,2' or 3,3' or 5,5' or 6,6' or 7,7' or 8,8' positions according to general formula (II) 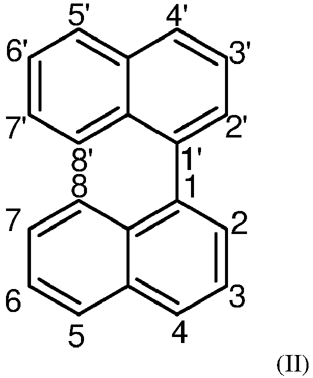
Wherein the remaining positions 2,2'; 3,3'; 4,4'; 5,5'; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COCl, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)ₓ(OCH₂CH₂)_{y}O(CH₂)_{z}CH₃, wherein x is an integer from 0 to 20; y is an integer from 0 to 50 and z is an integer from 0 to 20.

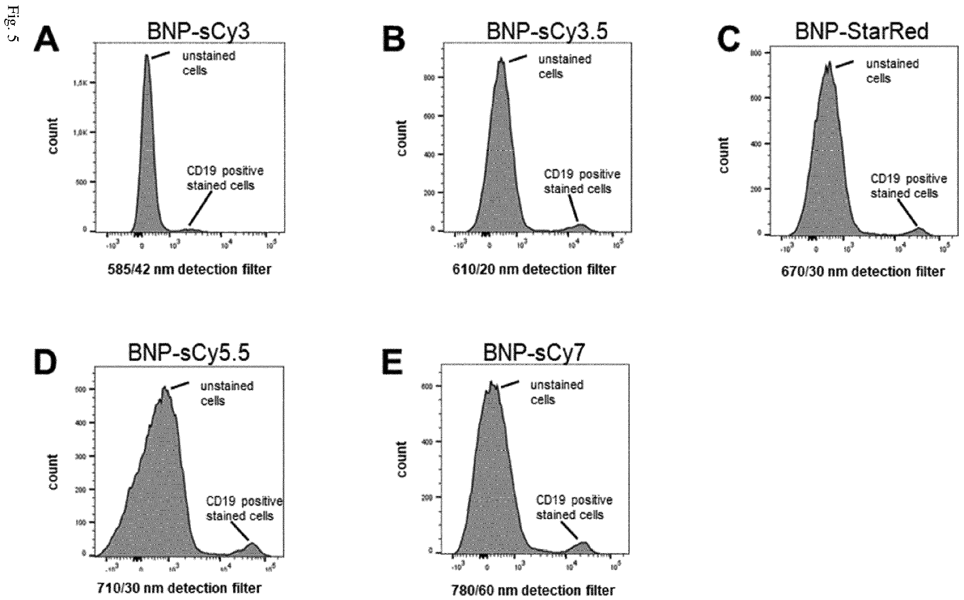

As can be seen from formula II, two positions out of 2,2'; 3,3'; 4,4'; 5,5'; 6,6'; 7,7' and 8,8' are needed for binding AR into the polymer chain. Accordingly, the term "remaining positions" refers to the 12 positions of AR that are not used for the binding of AR in the polymer chain and which are available for substitution with the residues as disclosed. Fig. 5 shows possible substitution patterns. The residues used for substitution may be same or different in each position (for example position 2 and 2' may be provided with the same or a different residue.

Optionally, two residues at the remaining positions may form a common cycloalkyl- or heterocycloalkyl ring system.

The term "AR is connected in the polymer chain via the 2,2' or 3,3' or 5,5' or 6,6' or 7,7' or 8,8' positions" refers to C-C bonds between the respective C atoms of the positions of AR with a C-atom of another AR unit, a G1 unit or an L unit.

Further object of the invention is a method for detecting a target moiety in a sample of biological specimens with at least one conjugate as disclosed herein characterized by the steps
a) contacting the sample of biological specimens with at least one conjugate, thereby labeling the target moiety recognized by an antigen recognizing moiety with the conjugate;
b) exciting the labelled target moieties with light having a wavelength within the absorbance spectrum of the conjugate
c) detecting the labelled target moieties by detecting the fluorescence radiation emitted by the conjugate.

Yet another object of the invention is the use of the method in fluorescence microscopy, flow cytometry, fluorescence spectroscopy, cell separation, pathology or histology.

### BRIEF DESCRIPTION OF THE DRAWINGS

Fig. 1 shows a scheme to demonstrate how the Poly-binaphthyl-co-monomer tandem system may be used to separately tune the excitation energy through the choice of modifying unit (MU)

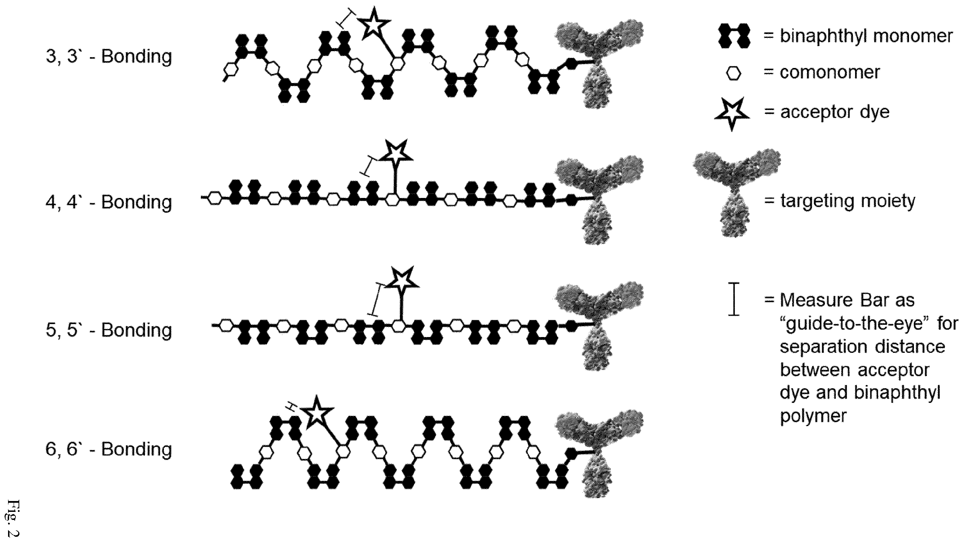

Fig. 2 shows examples of tandem binaphthyl comonomer polymer systems coupled to a targeting moiety. Depending on the chosen bonding between the binaphthyl monomers, the polymer may transition from predominantly linear to predominantly helical structure, which allows the tuning of energy transfer efficiency from the polymer donor to the acceptor dye.

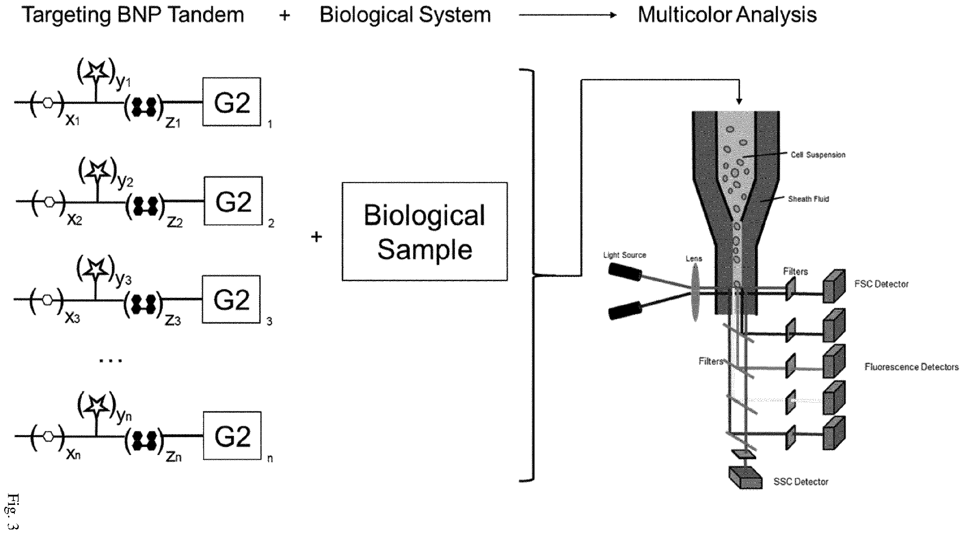

Fig. 3 shows example use of multiple different color binaphthyl-tandem dye systems conjugated to different targeting moieties being combined with a biological sample to for multiplexed analyte detection.
Fig. 4 a-f shows. the fluorescence resonance energy transfer from these compound according to the examples of the invention
Fig. 5 shows the performance of polymer dye monoclonal antibody (anti-CD19) conjugates according to the invention in flow cytometric analysis of peripheral blood mononuclear cells (PBMCs) with excitation at 355 nm is shown

### DETAILED DESCRIPTION

The general structure for binaphthyl (BNP) based water-soluble fluorescent π-conjugated polymer dyes as shown in formula (I) with both tunable absorption band and tunable emission wavelength. MU is a modifying unit for tuning the absorption band. Ar is the 1,1'-binaphthyl-based aryl unit. L is an optional linker, G1 and G2 are both terminal moieties. FL is a fluorescent dye for color-tunable emission. MU^{∗} is a modifying unit with a chemoselective functional group which can be used to selectively add a fluorescent dye for color-tunable emission.

In a variant on the invention, at least two positions 2,2'; 3,3'; 4,4'; 5,5'; 6,6' 7,7' and 8,8' are substituted with residues according to general formula (IV) 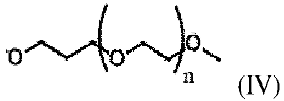 With n = 5 to 15

The conjugates of the invention are preferable water-soluble.

### MU and MU^{∗} as band gap modifying units

MU and MU^{∗} are bandgap modifying unit which cause a shift of the absorption band of the conjugate.

MU^{∗} is a bandgap modifying unit similar or identical to MU but may be connected to chemoselective moiety through any modifiable atom not used for polymerization. Chemoselective moieties include, but are not limited to: amine, carbamate, carboxylic acid, carboxylate, maleimide, activated ester, *N*-hydroxysuccinimidyl, hydrazine, hydrazide, hydrazine, azide, alkyne, aldehyde, thiol, thiocyanate, and protected groups thereof for conjugation to a fluorescent dye and fluorescence emission tuneability.

In order to cause a shift of the absorption band of the conjugate, MU and MU* may each comprise the same or different aromatic system with at least one benzene or thiophene ring providing an conjugated pi system, optionally substituted with an electron withdrawing residue, an electron donating residue, sterically hindering groups, and/or residues enhancing water solubility.

More preferable, MU and MU* comprise each the same or different aromatic system with at least two fused, annulated or conjugated benzene or thiophene rings providing an conjugated pi system, optionally substituted with an electron withdrawing residue, an electron donating residue, sterically hindering groups, and/or residues enhancing water solubility.

As electron withdrawing residue, an electron donating residue, sterically hindering groups, and/or residues enhancing water solubility one or more residues like alkyl or ether (glycol) residues or halogen atoms or halogenated alkyl groups can be used.

The MU and MU* repeating units may evenly or randomly distributed in the conjugate of the invention. Insofar, MU and MU* have not only the function as band gap modifying unit, but also as polymer modifying unit.

Preferred residues for MU and MU* (optionally substituted as disclosed) are as follows:

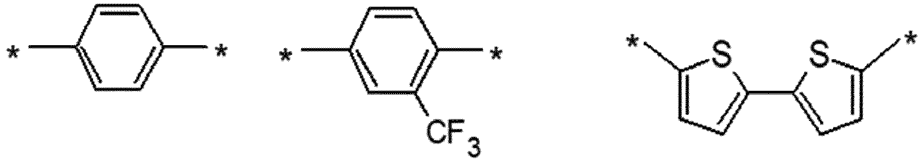

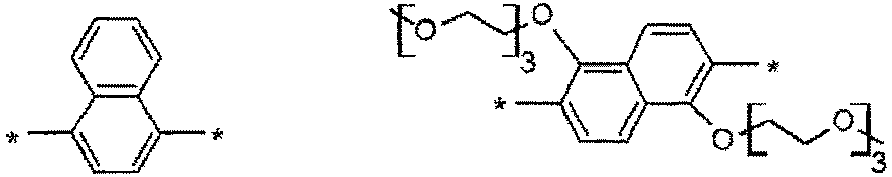

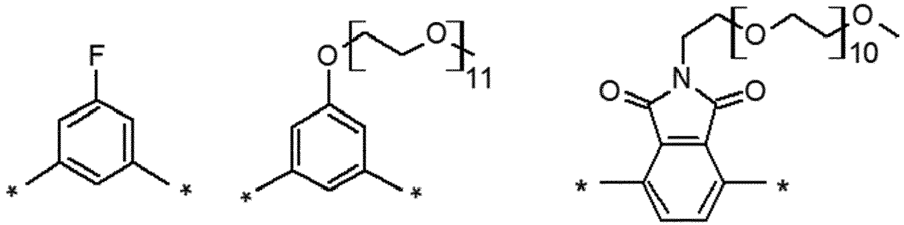

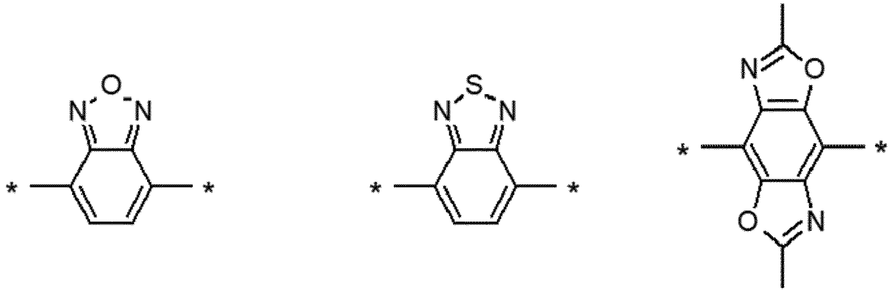

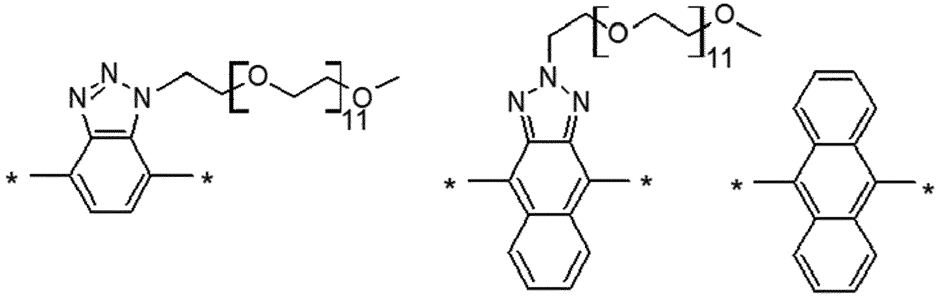

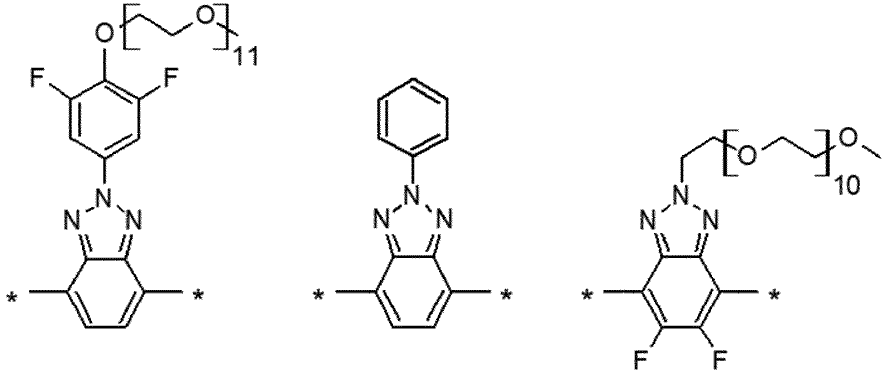

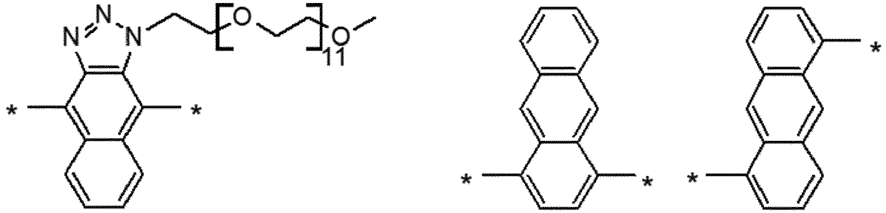

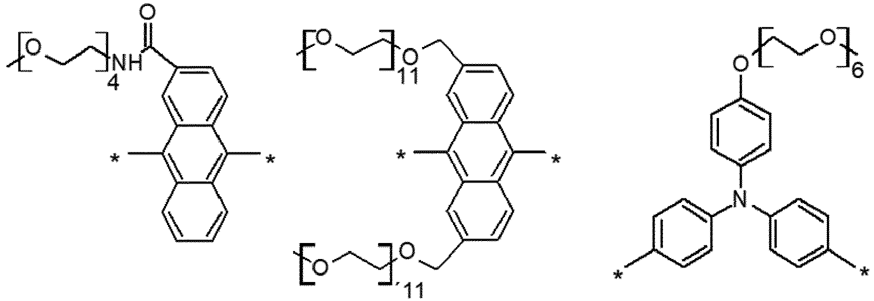

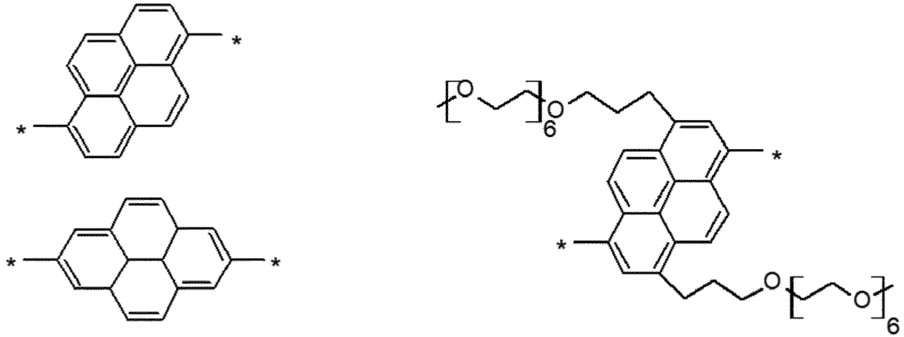

### Terminal groups G1 and G2

G1 and G2 are both independently chosen from the group of hydrogen, halogen or an antigen recognizing moiety. The antigen recognizing moiety could be selected from an antibody, an fragmented antibody, an fragmented antibody derivative, peptide/MHC-complexes, receptors for cell adhesion or costimulatory molecules, receptor ligands, antigens, hapten binders, avidin, streptavidin, aptamers, primers and ligase substrates, peptide/MHC complexes targeting TCR molecules, cell adhesion receptor molecules, receptors for costimulatory molecules or artificial engineered binding molecules.

### Fluorescent Moiety FL

Suitable fluorescent moieties FL are those known from the art of immunofluorescence technologies, e.g., flowcytometry or fluorescence microscopy. In the method of the invention, the target moiety labelled with the conjugate is detected by exciting the polymer backbone or the fluorescent moiety FL or both and detecting the resulting emission (photoluminescence) of FL or the polymer.

Useful fluorescent moieties FL might be protein based, such as phycobiliprotein, small organic molecule dyes, such as xanthenes, like fluorescein, or rhodamines, cyanines, oxazines, coumarins, acridines, oxadiazoles, pyrenes, pyrromethenes, pyridyloxazole or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

In a variant of the invention, the fluorophore FL is substituted with one more water solubility imparting substituents selected from the group consisting of sulfonates, phosphonates, phosphates, polyethers, sulfonamides and carbonates. It is particularly advantageous to use fluorescent moieties with sulfonate substituents, such as dyes of the Alexa Fluor family provided by Thermo Fisher Scientific Inc. The degree of sulfonate substitution per fluorophore may be 2 or more, i.e., for rhodamine dyes or cyanine dyes.

Suitable commercial available fluorescent moieties may be purchased from the product line "Vio" from Miltenyi Biotec BV & Co. KG, or FITC, or Promofluor, or Alexa Dyes and/or Bodipy dyes from Thermofisher, or Cyanines from Lumiprobe or DY^{™} Fluorophore from Dyomics GmbH or Star Dyes from Abberior GmbH.

### Linker group L

L is a linker which is comprised of an aryl or a heteroaryl group located on the ends of the polymer main chain and is substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) a conjugated organic dye as acceptor dye, or iii) a biomolecule.

Preferable, L is selected from one or more of the following residues:

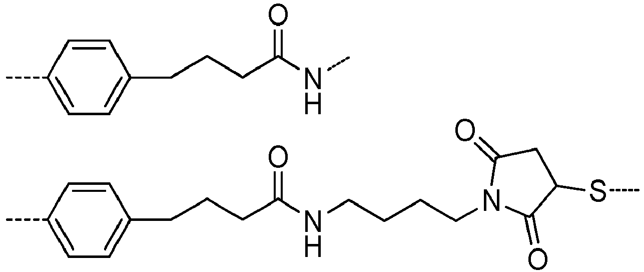

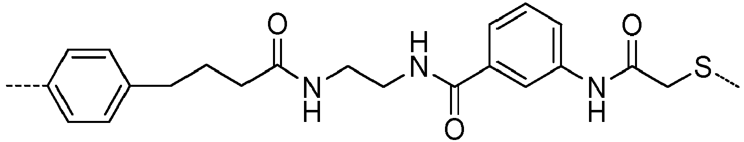

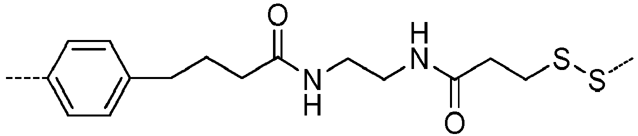

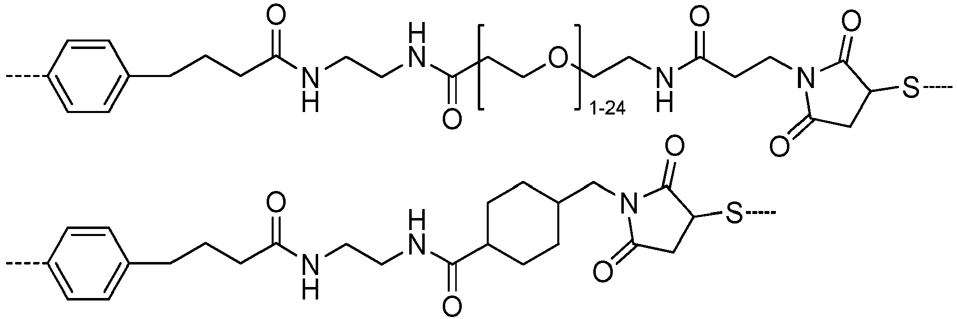

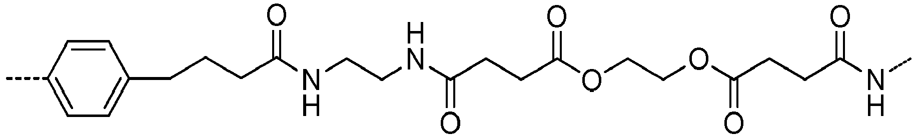

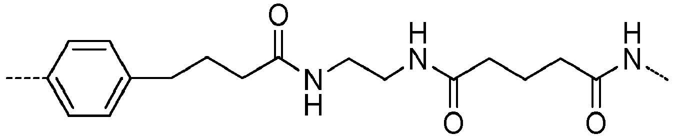

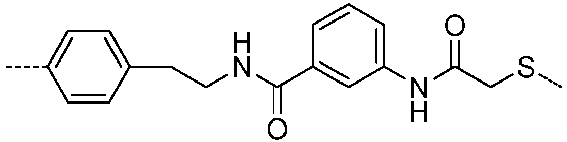

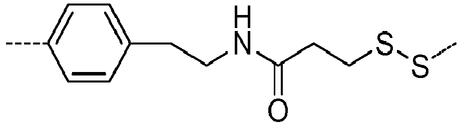

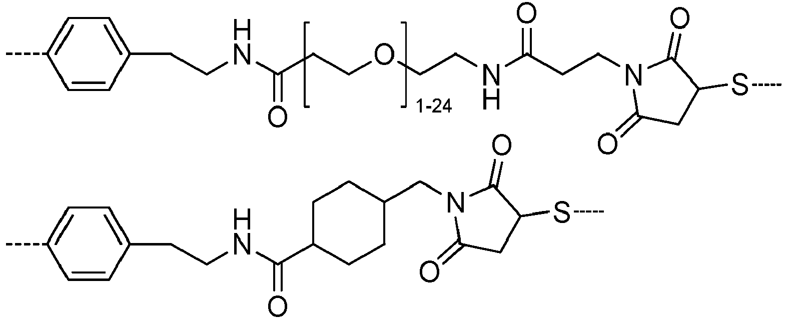

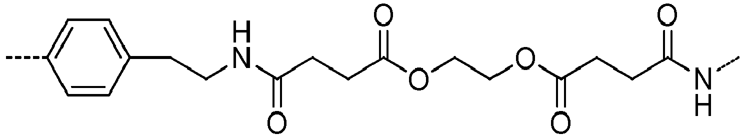

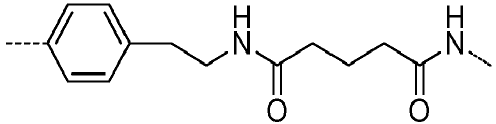

### Antigen recognizing moiety

The term "antigen recognizing moiety" refers to any kind of antibody, fragmented antibody or fragmented antibody derivatives, directed against the target moietiesexpressed on the biological specimens, like antigens expressed intracellular or extracellular on cells. The term relates to fully intact antibodies, fragmented antibody or fragmented antibody derivatives, e. g., Fab, Fab', F(ab')2, sdAb, scFv, di-scFv, nanobodies. Such fragmented antibody derivatives may be synthesized by recombinant procedures including covalent and non - covalent conjugates containing these kinds of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules.

The conjugate used in the method of the invention may comprise up to 100, preferable 1-20 antigen recognizing moieties Y. The interaction of the antigen recognizing moiety with the target antigen can be of high or low affinity. Binding interactions of a single low - affinity antigen recognizing moiety is too low to provide a stable bond with the antigen. Low-affinity antigen recognizing moieties can be multimerized by conjugation to the enzymatically degradable spacer to furnish high avidity. When the spacer is enzymatically cleaved, the low-affinity antigen recognizing moieties will be monomerized which results in a complete removal of the fluorescent marker.

Preferable, the term "Antigen recognizing moiety" refers to an antibody directed against antigen expressed by the biological specimens (target cells) intracellular, like IL2, FoxP3, CD154, or extracellular, like CD19, CD3, CD14, CD4, CD, CD25, CD34, CD56, and CD133. The antigen recognizing moieties G1, G2, especially antibodies, can be coupled to CP through side chain amino or sulfhydryl groups. In some cases, the glycosidic side chain of the antibody can be oxidized by periodate resulting in aldehyde functional groups.

The antigen recognizing moiety can be covalently or non-covalently coupled. Methods for covalent or non-covalent conjugation are known by persons skilled in the art and the same as mentioned for conjugation of the fluorescent marker.

The method of the invention is especially useful for detection and/or isolation of specific cell types from complex mixtures and may comprise more than one sequential sequences of the steps a) - c). The method may use a variety of combinations of conjugates. For example, a conjugate may comprise antibodies specific for two different epitopes, like two different anti-CD34 antibodies. Different antigens may be addressed with different conjugates comprising different antibodies, for example, anti-CD4 and anti-CD8 for differentiation between two distinct T-cell-populations or anti-CD4 and anti-CD25 for determination of different cell subpopulations like regulatory T-cells.

### Cell Detection Methods

Targets labelled with the conjugate are detected by exciting either the fluorescent moiety FL or the backbone CP and analysing the resulting fluorescence signal. The wavelength of the excitation is usually selected according to the absorption maximum of the fluorescent moiety FL or CP and provided by LASER or LED sources as known in the art. If several different detection moieties FL are used for multiple colour/parameter detection, care should be taken to select fluorescent moieties having not overlapping absorption spectra, at least not overlapping absorption maxima. In case of fluorescent moieties the targets may be detected, e.g., under a fluorescence microscope, in a flow cytometer, a spectrofluorometer, or a fluorescence scanner. Light emitted by chemiluminescence can be detected by similar instrumentation omitting the excitation.

### Target Moiety

The target moiety to be detected with the method of the invention can be on any biological specimen, like tissues slices, cell aggregates, suspension cells, or adherent cells. The cells may be living or dead. Preferable, target moieties are antigens expressed intracellular or extracellular on biological specimen like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo Sapiens).

### Use of the Method

The method of the invention can be used for various applications in research, diagnostics and cell therapy, like in fluorescence microscopy, flow cytometer, fluorescence spectroscopy, cell separation, pathology or histology.

In a first variant of the invention, biological specimens like cells are detected for counting purposes i.e. to establish the amount of cells from a sample having a certain set of antigens recognized by the antigen recognizing moieties of the conjugate. In another variant, the biological specimens detected by the conjugate in step c) are separated from the sample by optical means, electrostatic forces, piezoelectric forces, mechanical separation or acoustic means.

In another variant of the invention, the location of the target moieties like antigens on the biological specimens recognized by the antigen recognizing moieties of the conjugate is determined. Such techniques are known as "Multi Epitope Ligand Cartography", "Chip-based Cytometry" or "Multiomyx" and are described, for example, in EP0810428, EP1181525, EP 1136822 or EP1224472. In this technology, cells are immobilized and contacted with antibodies coupled to fluorescent moiety. The antibodies are recognized by the respective antigens on the biological specimen (for example on a cell surface) and after removing the unbound marker and exciting the fluorescent moieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties.

The location of the target moieties is achieved by a digital imaging device with a sufficient resolution and sensitivity in for the wavelength of the fluorescence radiation, the digital imaging device may be used with or without optical enlargement for example with a fluorescence microscope. The resulting images are stored on an appropriate storing device like a hard drive, for example in RAW, TIF, JPEG, or HDF5 format.

In order to detect different antigens, different antibody-conjugates having the same or different fluorescent moiety or antigen recognizing moiety can be provided. Since the parallel detection of fluorescence emission with different wavelengths is limited, the antibody-fluorochrome conjugates are utilized sequentially individually or in small groups (2-10) after the other.

In yet another variant of the method according to the invention, the biological specimens especially suspension cells of the sample are immobilized by trapping in microcavities or by adherence.

In general, the method of the invention can be performed in several variants. For example, the conjugate not recognized by a target moiety can be removed by washing for example with buffer before the target moiety labelled with the conjugate is detected.

In a variant of the invention, at least two conjugates are provided simultaneously or in subsequent staining sequences, wherein each antigen recognizing moiety recognizes different antigens. In an alternative variant, at least two conjugates can be provided to the sample simultaneously or in subsequent staining sequences, . In both cases, the labelled target moieties can be detected simultaneously or sequentially.

### EXAMPLES

By way of example following compound was synthesized in the following general method with different acceptor dyes FL and conjugated to antigen recognizing moieties.

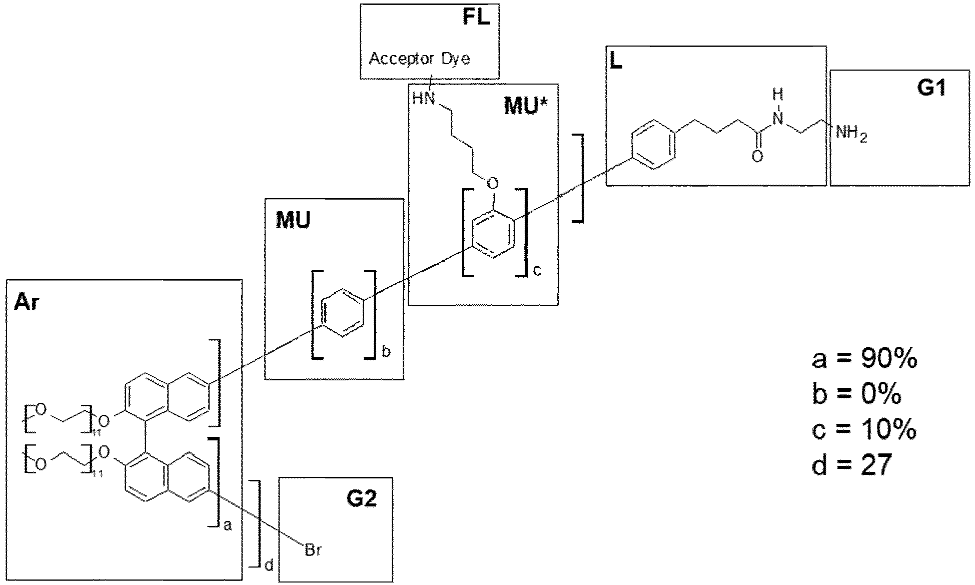

| **Polymer- Acceptor Dye** | **l*_{abs}*** / **nm** | **l*ₑₘ* / nm** | ***QY (exc325)*** / % | **I_{donor}/I_{acceptor}** |
|---|---|---|---|---|
| BNP-Fluorescein | 264 | 530 | 2.8 | 1.50 |
| BNP-sCy3 | 264 | 576 | 9.7 | 1.20 |
| BNP-sCy3.5 | 264 | 605 | 35.5 | 0.25 |
| BNP-sCy5 | 264 | 678 | 21,1 | 0.33 |
| BNP-sCy5.5 | 264 | 706 | 25,5 | 0.51 |
| BNP-sCy7 | 264 | 788 | 8,8 | 0.58 |
| BNP-Star Red | 264 | 662 | 26,5 | 0.56 |

The table shows physical data of different polymer-acceptor dye tandem systems. The poly(binaphthyl-co-phenylene-NH2) polymer as shown above was coupled to the different acceptor dyes FL and the fluorescence emission properties measured to determine emission wavelength (lem), fluorescence quantum yield (QY), and efficiency of energy transfer (Idonor/Iacceptor). All measurements were performed by exciting the binaphthyl polymer at 325 nm.

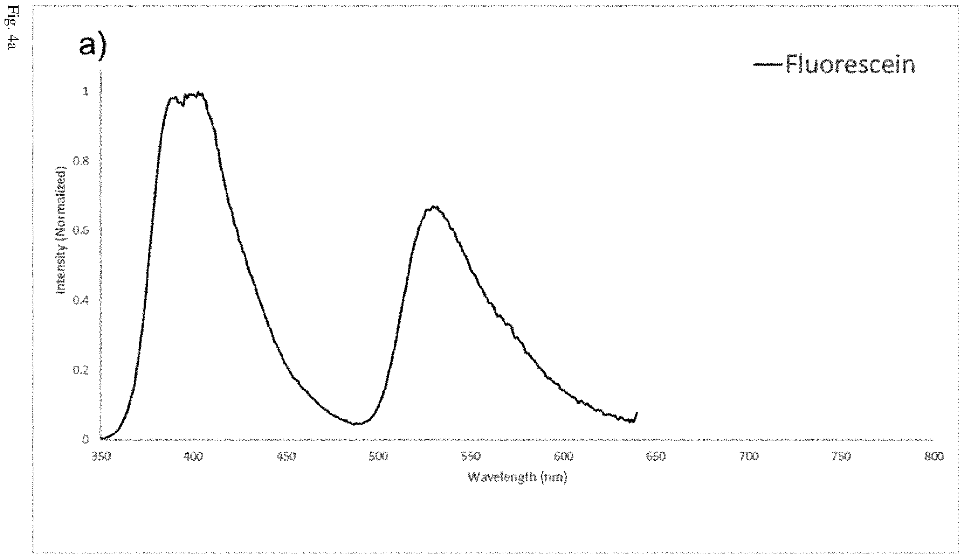

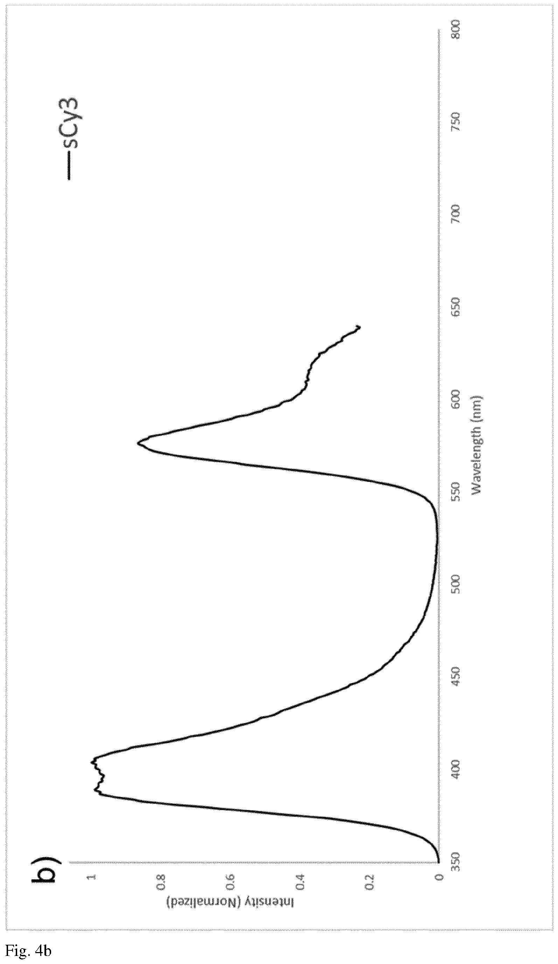

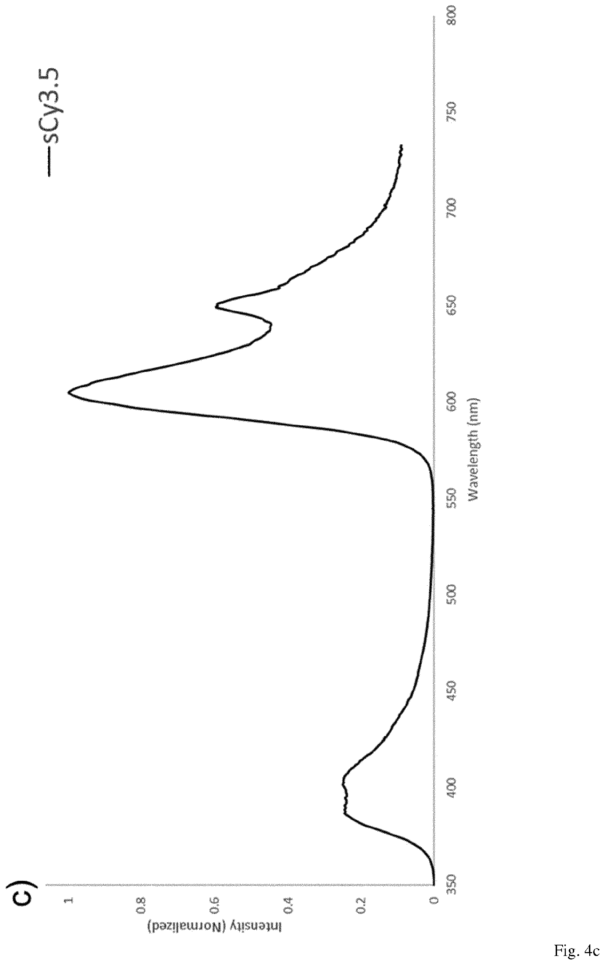

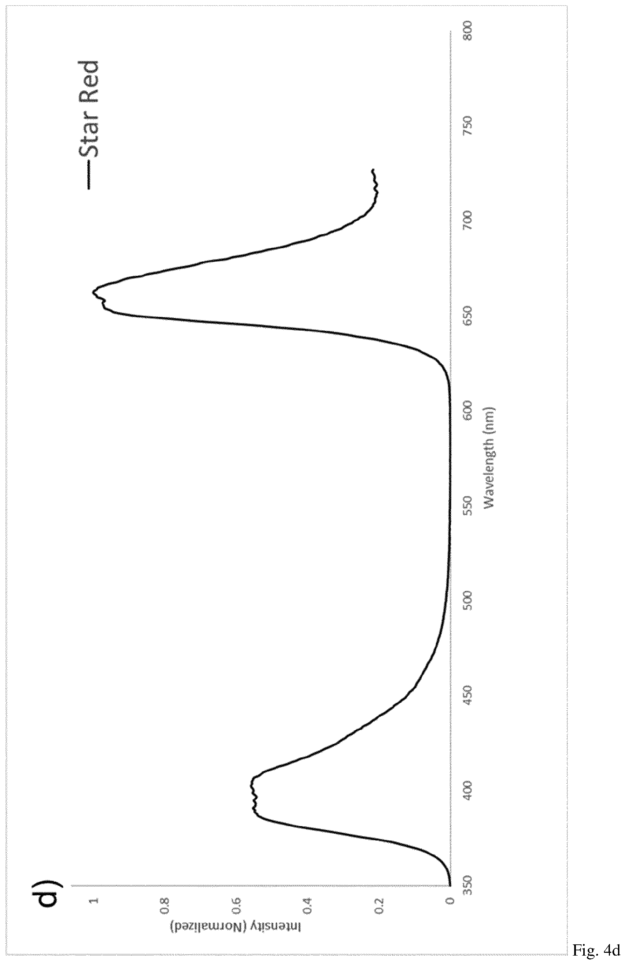

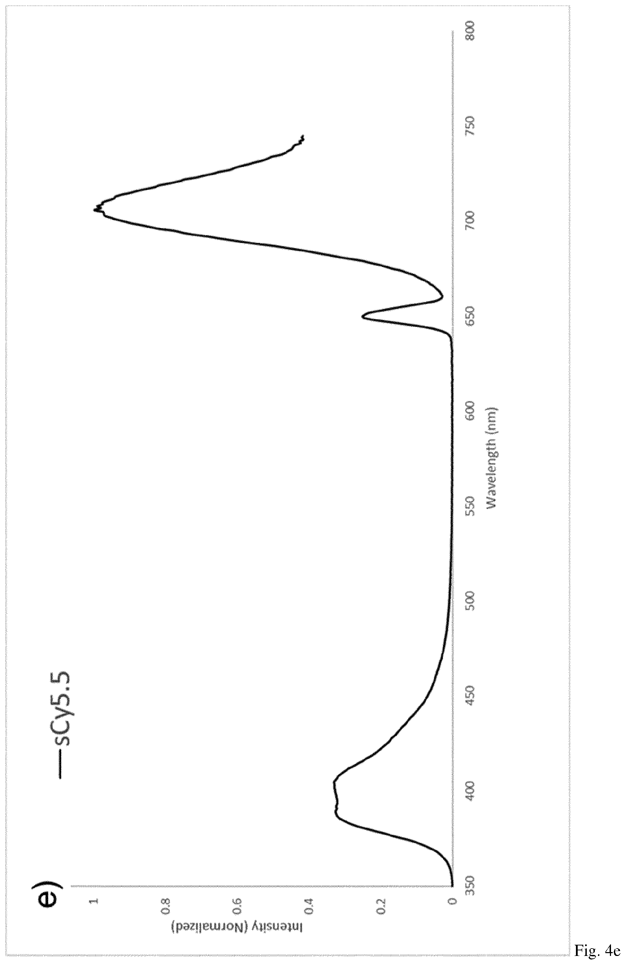

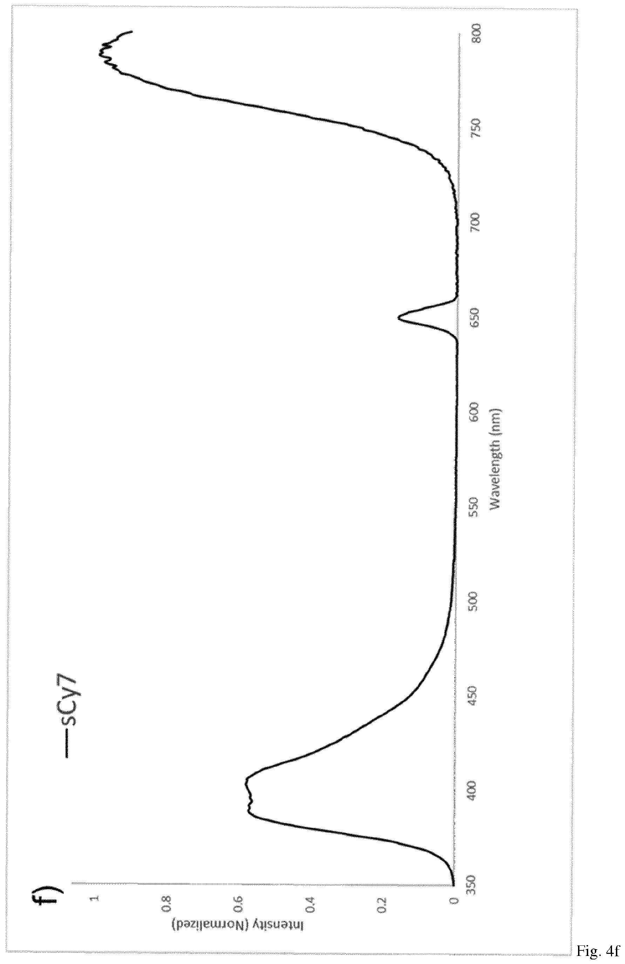

The acceptor dyes used are the known sulfo-cyanines and Star Red dyes. Fig. 4 a-f shows . the fluorescence resonance energy transfer from these compounds. Binaphthyl residual donor emission is centered around 400 nm in each plot. The fluorescence emission via energy transfer through acceptor dyes is for a) Fluorescein (-530 nm), **b)** Sulfo-Cy3 (-576 nm), **c)** Sulfo-Cy3.5 (-605 nm), **d)** Sulfo-Cy5.5 (-706 nm), e) Sulfo-Cy7 (-788 nm) and **f)** Star Red (-662 nm). The sharp emission feature centered around 650 nm in graphs c, d, e, and f is an artifact of the emission measurement and is not a real feature of the tandem polymer fluorescence.

The performance test of polymer dye monoclonal antibody (anti-CD19) conjugates in flow cytometric analysis of peripheral blood mononuclear cells (PBMCs) with excitation at 355 nm is shown in Fig. 5. Only CD14 negative cells are shown to exclude monocytes for clarity. A) BNP-sCy3 conjugate detected with 585/42 nm emission filter; B) BNP-sCy3.3 conjugate detected with 610/20 nm emission filter; C) BNP-Star Red conjugate detected with 670/30 nm emission filter; D) BNP-sCy5.5 conjugate detected with 710/30 nm emission filter; E) BNP-sCy7 conjugate detected with 780/60 nm emission filter.

### General Synthesis

### Synthesis of building block M1

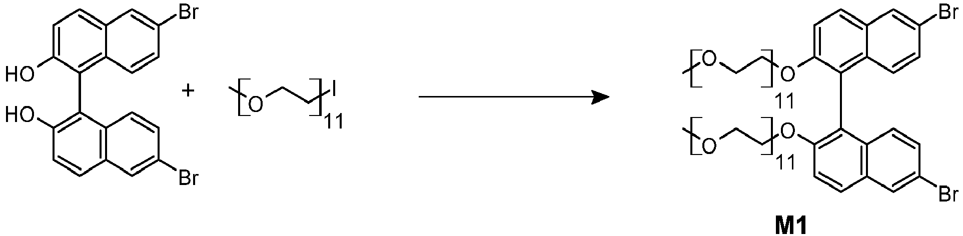

To a stirred solution of 6,6'-dibromo-[1,1'-binaphthalene]-2,2'-diol (3.00 g, 6 mmol) in acetone (42 mL), was added 34-iodo-2,5,8,11,14,17,20,23,26,29,32-undecaoxa tetratriacontane (12.6 g, 20 mmol), followed by K2CO3 (2.82 g, 20 mol), and the resulting reaction mixture was stirred at 70 °C for 16 h. The reaction mixture was filtered and concentrated under reduced pressure to dryness. The obtained crude material was diluted with water (20 mL) and extracted with ethyl acetate (3 × 30 mL). The combined organic phases were washed with brine (20 mL), dried over Na2SO4, filtered and concentrated under reduced pressure. The obtained crude residue was purified by silica column chromatography eluting with methanol/dichloromethane (5:95) giving a pale-yellow liquid. This compound was further purified by reverse phase chromatography, using eluting with acetonitrile/water (50:50). The compound was freeze dried to give pure compound **M1** (1.7 g, 17.6 %) as a pale-yellow liquid.

### Synthesis of building block M2

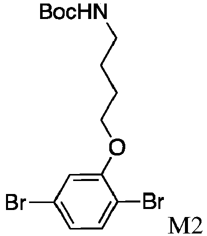

Potassium carbonate (2.74 g, 19.9 mmol) was added to a solution of 2,5-dibromophenol (1.00 g, 3.97 mmol) in dimethylformamide (40 mL). 4-(N-Boc-amino)butyl bromide (1.2 g, 4.8 mmol, 1.2 eq.) was added and the solution was heated to 80 °C for 72 h. The solvent was removed under reduced pressure and the residue was suspended in dichloromethane (50 mL) and washed with water (3 × 50 mL). The organic phase was dried over sodium sulfate and the solvent was removed under reduced pressure to give a white solid (1.49 g). The crude product was purified by silica flash column chromatography with a gradient of 0 to 50 vol-% ethyl acetate in n-hexane, followed by reversed-phase flash column chromatography with a gradient of 0 to 100 vol-% acetonitrile in water. The product was obtained as a white solid (1.46 g, 87 %).

### Synthesis of tert-butyl 4-(4-bromophenyl)butanoate (M3)

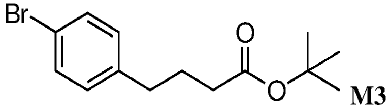

1,1'-Carbonyldiimidazole (667 mg, 4.11 mmol) was added to a solution of 4-(4-bromophenyl)butanoic acid (1.00 g, 4.11 mmol) in dimethylformamide (4.1 mL) and the mixture was stirred at 40 °C for 1 h. *t*-Butyl alcohol (610 mg, 772 µL, 8.23 mmol) and 1,8-diazabicyclo [5.4.0]undec-7-en (626 mg, 614 µL, 4.11 mol) were added and the mixture was stirred at 40 °C for 24 h. Diethyl ether (50 mL) was added and the solution was washed with 10 % hydrochloric acid (10 mL), water (10 mL) and 10 % aq. sodium carbonate (10 mL). The organic phase was dried over sodium sulfate and concentrated under reduced pressure. The crude product was purified by silica flash column chromatography eluting with a gradient of 0 to 10 vol-% ethyl acetate in n-hexane to yield the product as a colorless oil (234 mg, 17 %).

### Synthesis of polymer backbone

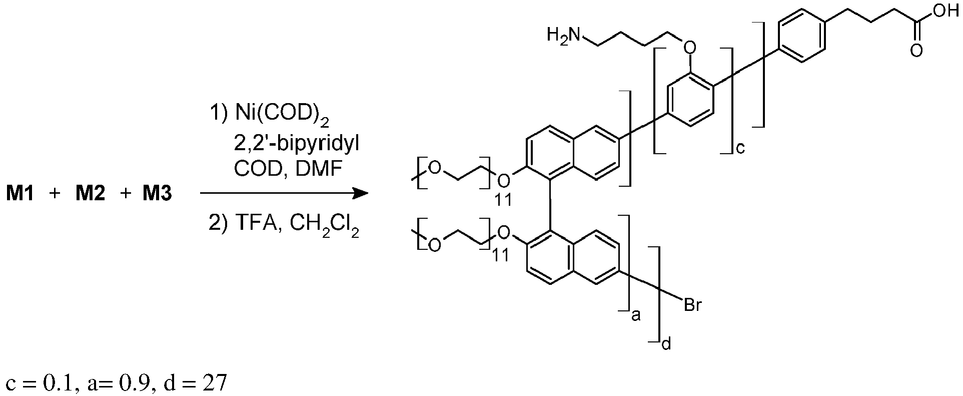

Bis(1,5-cyclooctadiene)nickel(0) (117 mg, 424 µmol), 2,2'-bipyridyl (66.0 mg, 424 µmol) and 1,5-cyclooctadiene (46.0 mg, 424 µmol) were dissolved in dimethylformamide (8 ml) and stirred at 70 °C for 30 min under argon atmosphere. The monomers **M1** (250 mg, 173 µmol), **M2** (8 mg, 19 µmol) and **M3** (1.1 mg, 3.9 µmol) were dissolved in dimethylformamide (8 mL) under argon atmosphere and added to the Ni-complex containing solution. The mixture was stirred for 3 h at 70 °C. The solvent was removed under reduced pressure, and the residue was suspended in 20 vol-% aq. ethanol. After centrifugation the supernatant was purified using size exclusion filtration and subsequently freeze-dried to yield the polymer as a yellow foam. The polymer was dissolved in dichloromethane (50 mL) and trifluoroacetic acid (5 mL) was added. The solution was stirred for 2 h at room temperature. Volatiles were removed under reduced pressure, and the residue was dissolved in 20 vol-% aq. ethanol (100 mL), purified by size exclusion filtration (10 kDa cutoff) and freeze dried to yield the final product (122 mg, 44 %).

### Synthesis of polymer tandem dyes

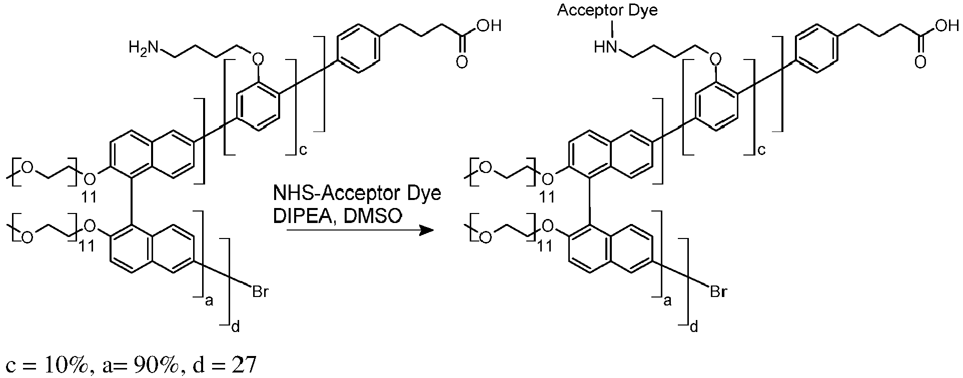

To a solution of polymer backbone (5.0 mg) in DMSO (0.33 µL) was added a solution of NHS-acceptor dye (1.128 µmol) in DMSO (0.1 - 0.7 mL). *N,N*-Diisopropylethylamine (10.8 µL) was added and the mixture was stirred at room temperature overnight. The reaction mixture was poured into 20 vol-% EtOH (40 mL). The excess of acceptor dye was removed by ultrafiltration. The polymer was purified by repeating a sequence of dissolving in 20 vol-% EtOH (15 mL) and concentration by ultrafiltration until the filtrate was colorless.

### Amination of tandem dyes

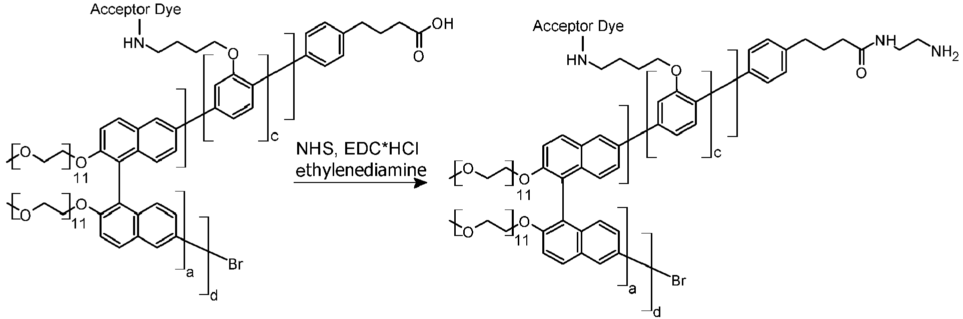

The polymer tandem dye (5.0 mg) was dissolved in MES buffer (50 mM, pH 6) containing 20% vol-% ethanol (0.50 mL). A solution of *N*-Hydroxysuccinimide (2.3 mg, 20 µmol) in 50 mM MES buffer (192 µL) and a solution of 1-ethyl-3-(3-dimethylaminopropyl)-carbodiimide hydrochloride (3.8 mg, 20 µmol) in 50 mM MES buffer (190 µL) were added. The mixture was stirred for 20 min at room temperature. Ethylenediamine (1.73 µL, 1.56 mg, 26 µmol) was added and the mixture was stirred for 2 h at room temperature. The mixture was diluted with 50 mM MES buffer (10 mL) and concentrated by ultrafiltration. The polymer was purified by dissolving in 20 vol-% EtOH (15 mL) followed by ultrafiltration and freeze drying.

### Bioconjugation of polymer tandem dyes to antibodies

The antibody was reduced using dithiothreitol (50 mM) in MES buffer (pH 6) for 30 min at an antibody concentration of 5 mg/mL. The reduced antibody was purified by gel filtration oven a Sephadex G-25 column (Cytiva Europe GmbH, Germany). The antibody containing fractions were identified by Coomassie stain and the antibody concentration of the pooled fractions was determined by measuring absorption at 280 nm.

The amino-functionalized polymer was coupled to the reduced antibody using succinimidyl 4-(N-maleimidomethyl)cyclohexane-1-carboxylate (SMCC) as heterobifunctional crosslinker. The cross-coupling was carried out using a commercial SMCC-cross-coupling kit (Thermo Fisher Scientific Inc., USA). The coupling was carried out following the kit user instructions. The antibody-polymer conjugates were purified by size exclusion chromatography.

### Cell Staining

Cells were washed by vortexing at a concentration of 1 × 10⁷ cells per mL using PEB buffer and subsequent centrifugation at 300 g for 5 min. 1 × 10⁷ cells were resuspended in 20 µL PEB buffer, the antibody-dye conjugate and PEB buffer were added to reach a concentration of 1 × 10⁷ cells per mL for the cell staining. The cells were stained by incubation for 10 min at 4 °C. Afterwards the cells were washed again and resuspended in 1 mL PEB buffer for flow cytometric analysis. The stained cells were stored at 4 °C until analysis. For dead cell discrimination, propidium iodide solution (titer 1:1000) was added by the instrument immediately before the measurement.

### Flow Cytometric Analysis

Stained cells were analyzed by flow cytometry using a LSR Fortessa (Becton, Dickinson & Company Inc, USA) with five laser excitation sources (355 nm, 405 nm, 488 nm, 561 nm, 640 nm) in combination with a 379/28 nm bandpass filter. Flow cytometry data analysis was performed using FlowJo (Becton, Dickinson & Company Inc, USA) flow cytometry analysis software.

## Claims

1. A conjugate having the general formula (I) 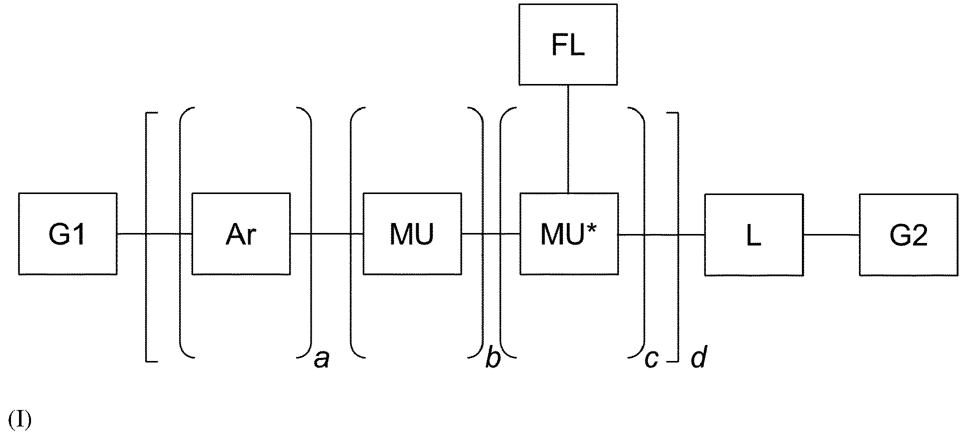
Wherein AR, MU and MU* are repeating units of a polymer and MU and MU* are polymer modifying units or band gap modifying units which are evenly or randomly distributed along the polymer main chain,
G1 and G2 stand for hydrogen, halogen or an antigen recognizing moiety, with the provision that at least one of G1 or G2 is an antigen recognizing moiety,
a is 10 to 100 mol%,
b is 0 to 90 mol%
c is 0.1 to 90 mol%
d is 1 to 10 000; with the provisio that a + b + c = 100 mol%
**characterized in that** AR is connected in the polymer chain via the 2,2' or 3,3' or 4,4' or 5,5' or 6,6' or 7,7' or 8,8' positions according to general formula (II) 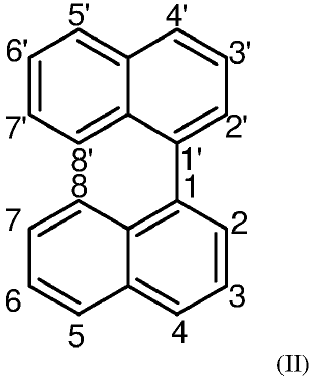
Wherein the remaining positions 2,2'; 3,3'; 4,4'; 5,5'; 6,6'; 7,7' and 8,8' are substituted with same or different residues selected from the group consisting of H, SO₂CF₃, SO₂Rₐ, CF₃, CCl₃, CN, SO₃H, NO₂, NRₐR_{b}R_{c}⁺, CHO, CORa, CO₂Ra, COCl, CONRaRb, F, Cl, Br, I, Rₐ, ORₐ, SRₐ, OCORₐ, NRₐR_{b}, NHCORₐ, CCRₐ, aryl-, heteroaryl-, C₆H₄ORₐ or C₆H₄NRaRb, with Ra-c independently hydrogen, alkyl-, alkenyl-, alkinyl-, heteroalkyl-, aryl-, heteroaryl-, cycloalkyl-, alkylcycloalkyl-, heteroalkylcycloalkyl-, heterocycloalkyl-, aralkyl- or a heteroaralkyl residue or (CH₂)ₓ(OCH₂CH₂)_{y}O(CH₂)_{z}CH₃, wherein x is an integer from 0 to 20; y is an integer from 0 to 50 and z is an integer from 0 to 20.

2. Conjugate according to claim 1 **characterized in that** the conjugate has a main chirality with AR provided mainly as *R*- or *S*-stereo isomer.

3. Conjugate according to claim 1 or 2, **characterized in that** the antigen recognizing moiety is selected from the group consisting of an antibody, an fragmented antibody, an fragmented antibody derivative, peptide/MHC-complexes, receptors for cell adhesion or costimulatory molecules, receptor ligands, antigens, hapten binders, avidin, streptavidin, aptamers, primers and ligase substrates, peptide/MHC complexes targeting TCR molecules, cell adhesion receptor molecules, receptors for costimulatory molecules or artificial engineered binding molecules.

4. Conjugate according to any of the claims 1 to 3, **characterized in that** MU and/or MU* comprise the same or different aromatic system with at least one benzene or thiophene ring providing an conjugated pi system.

5. Conjugate according to any of the claims 1 to 4, **characterized in that** MU and/or MU* are independently selected from one or more of the following residues

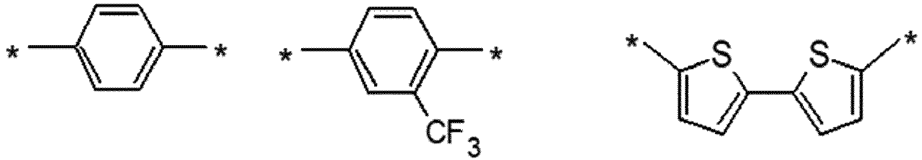

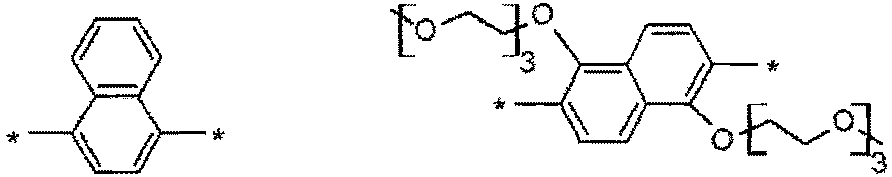

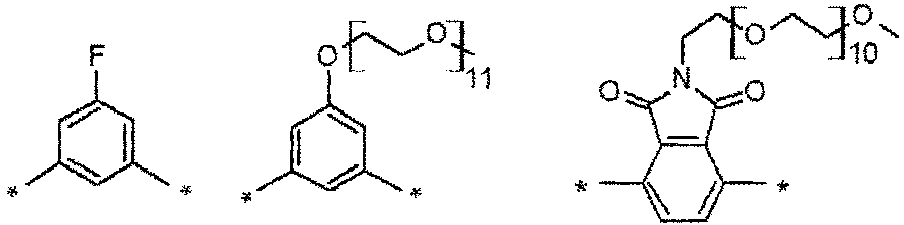

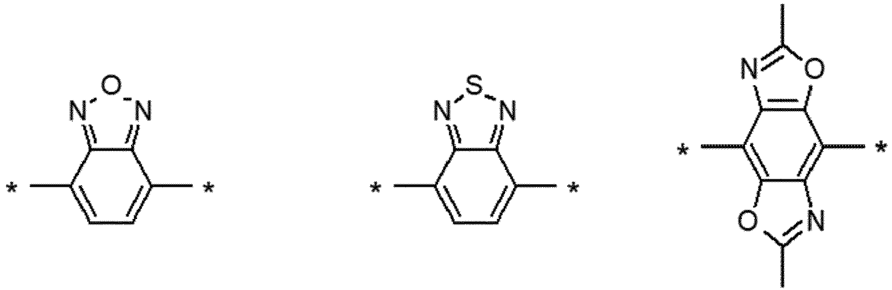

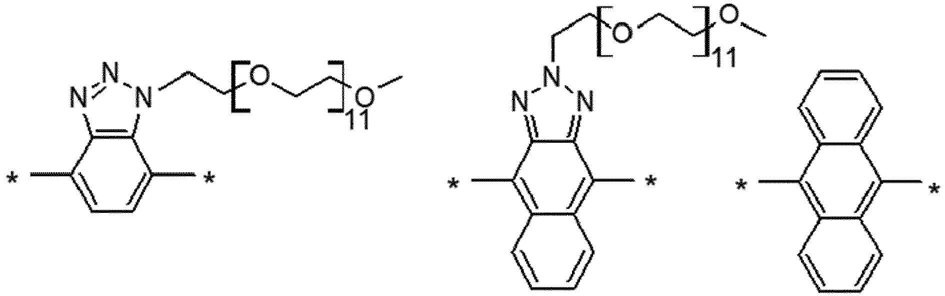

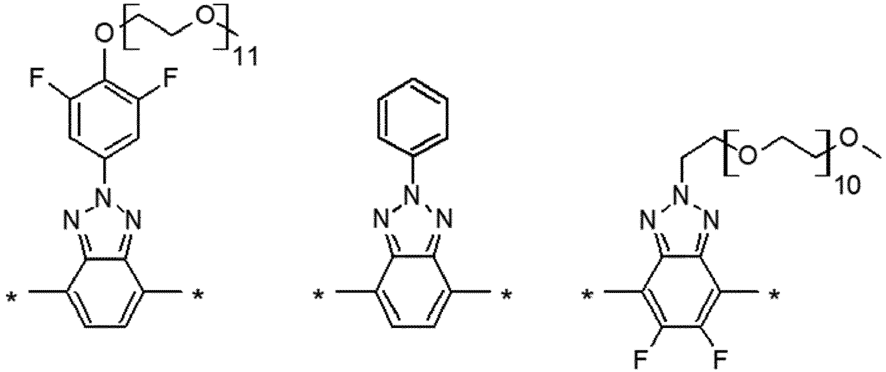

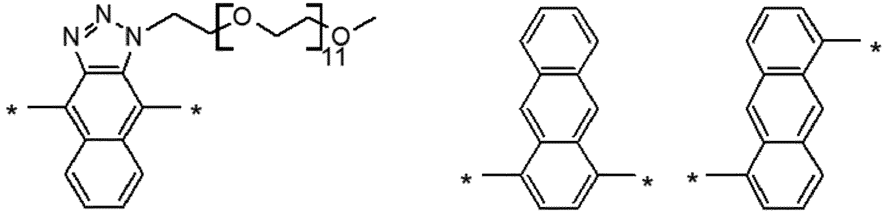

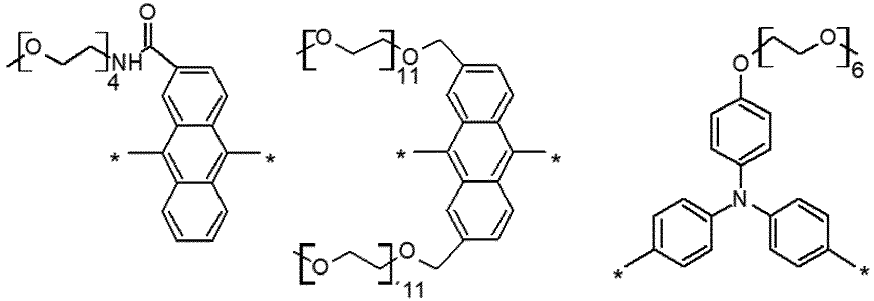

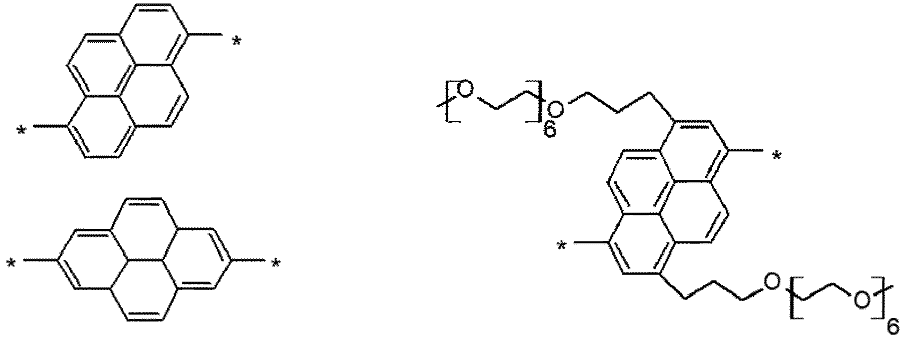

6. Conjugate according to any of the claims 1 to 5, **characterized in that** L is an aryl or a heteroaryl group located at the ends of the polymer main chain and may be substituted with one or more pendant chains terminated with: i) a functional group selected from amine, carbamate, carboxylic acid, carboxylate, maleimide, activated ester, N-hydroxysuccinimidyl, hydrazine, hydrazide, hydrazine, azide, alkyne, aldehyde, thiol, and protected groups thereof for conjugation to a molecule or biomolecule; or ii) an attached organic fluorescent dye as energy acceptor of an energy transfer system, or iii) a biomolecule.

7. The conjugate according to claim 6, **characterized in that** L is selected from one or more of the following residues:

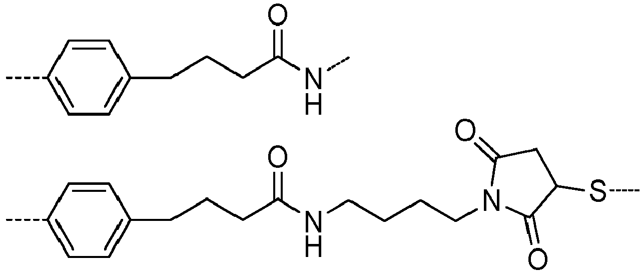

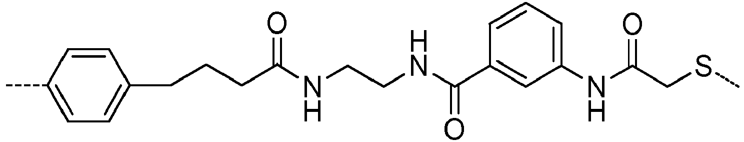

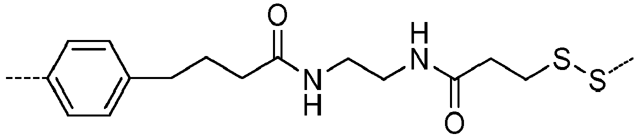

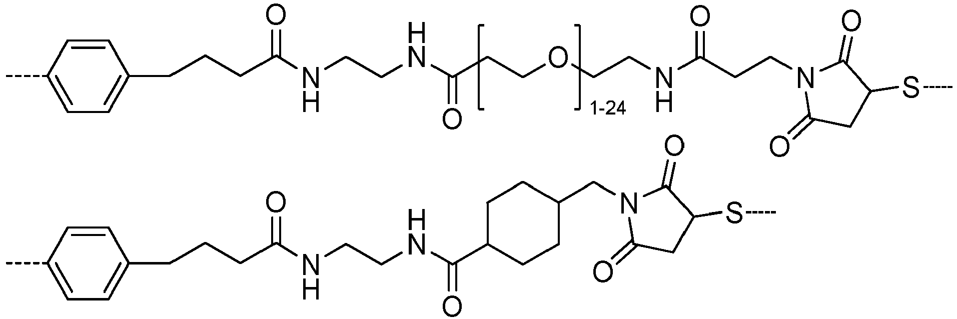

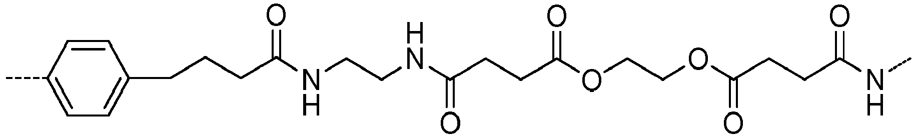

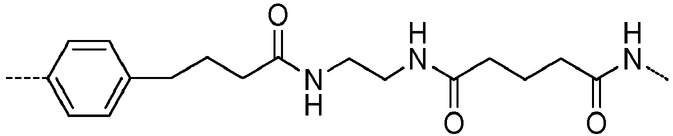

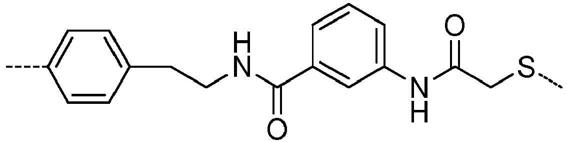

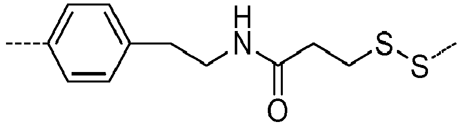

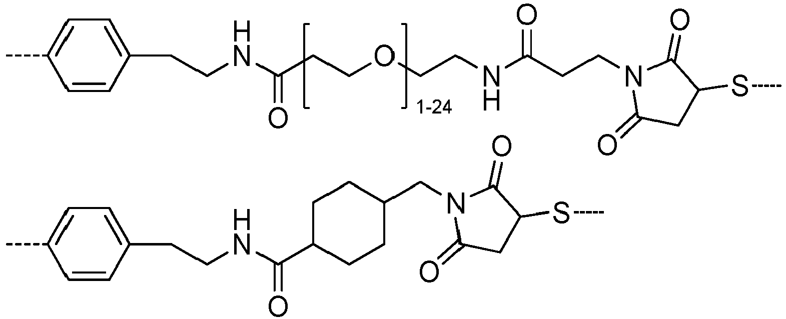

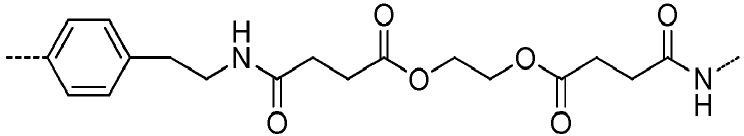

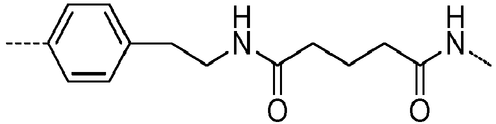

8. Conjugate according to any of the claims 1 to 7, **characterized in that** at least two positions 2,2'; 3,3'; 4,4'; 5,5'; 6,6' 7,7' and 8,8' are substituted with residues according to general formula (III) 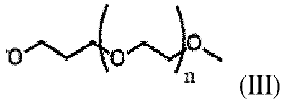 With n = 5 to 15

9. Conjugate according to any of the claims 1 to 8, **characterized in that** FL is selected from the group consisting of Fluorescein, Fluorescein-Derivatives, Rhodamine, Tetramethylrhodamine, Silicon-Rhodamine (SiR), Coumarines, Resorufines, Pyrenes, Anthracenes, Phenylenes, Phthalocyanines, Cyanines, Xanthenes, Amidopyrylium-Fluorophores, Oxazine, Quadrain-Farbstoffe, Carbopyronine, 7-Nitrobenz-2-Oxa-1,3-Diazol (NBD) Fluorophore, BODIPY^{™} Fluorophores (Molecular Probes, Inc.), ALEXA^{™} Fluorophore (Molecular Probes, Inc.), DY^{™} Fluorophores (Dyomics GmbH), Benzopyrylium Fluorophores, Benzopyrylium-Polymethine Fluorophores, Lanthanid-Chelate, Metalloporhyrines, Rhodol dyes, Carborhodol dyes, Naphthalimides und Porphyrines.

10. Method for detecting a target moiety in a sample of biological specimens with at least one conjugate according to any of the claims 1 to 9 **characterized by** the steps
d) contacting the sample of biological specimens with at least one conjugate, thereby labeling the target moiety recognized by an antigen recognizing moiety with the conjugate;
e) exciting the labelled target moieties with light having a wavelength within the absorbance spectrum of the conjugate
f) detecting the labelled target moieties by detecting the fluorescence radiation emitted by the conjugate.

11. Use of the conjugate according to any of the claims 1 to 9 in fluorescence microscopy, flow cytometer, fluorescence spectroscopy, cell separation, pathology or histology.
